# EUROPEAN PATENT SPECIFICATION

(11) **EP 4 263 522 B1**
(45) Date of publication and mention of the grant of the patent: **02.10.2024**
(21) Application number: 21840512.4
(22) Date of filing: 16.12.2021
(51) Int. Cl.: C07D 401/14, A61K 31/454, A61P 25/00

(54) **DIHYDROISOQUINOLINYL DERIVATIVES**
DIHYDROISOCHINOLINYLDERIVATE
DÉRIVÉS DE DIHYDROISOQUINOLÉINE

(30) Priority: 18.12.2020 EP 20215251
(43) Date of publication of application: 25.10.2023
(73) Proprietor: UCB Biopharma SRL, 1070 Brussels (BE)
(72) Inventor: HALL, Adrian, 1070 Brussels (BE)
(74) Representative: UCB Intellectual Property
(86) International application number: PCT/EP2021/086131
(87) International publication number: WO 2022/129303

(56) References cited:
- WO-A1-2014/193781
- WO-A1-2016/055479
- WO-A1-2017/178377
- WO-A1-2019/204418

## Description

The invention relates to dihydroisoquinolinyl derivatives and said derivatives for use in therapy. In particular the present invention relates to pharmacologically active fused dihydroisoquinolinyl derivatives and analogs thereof. More particularly, the present invention relates to substituted 3,4-dihydro-1H-isoquinolin-2-yl derivatives and analogs thereof.

The compounds according to the present invention are D1 Positive Allosteric Modulators and accordingly of benefit as pharmaceutical agents for the treatment of diseases in which D1 receptors play a role.

The monoamine dopamine acts via two families of GPCRs to modulate motor function, reward mechanisms, cognitive processes and other physiological functions. Specifically, dopamine is acting upon neurons via D1-like, comprising dopamine D1 and D5, receptors which couple mainly to the Gs G-protein and thereby stimulate cAMP production, and D2-like, which comprise D2, D3 and D4, receptors which couple to Gi/qG-proteins and which attenuate cAMP production. These receptors are widely expressed in different brain regions. In particular, D1 receptors are involved in numerous physiological functions and behavioural processes. D1 receptors are, for instance, involved in synaptic plasticity, cognitive function and goal-directed motor functions, but also in reward processes. Due to their role in several physiological/neurological processes, D1 receptors have been implicated in a variety of disorders including cognitive and negative symptoms in schizophrenia, cognitive impairment related to neuroleptic therapy, Mild Cognitive Impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction sleep disorders, apathy, traumatical spinal cord injury or neuropathic pain.

It has proven difficult to develop orally bioavailable small molecules targeting D1 receptors. D1 agonists developed so far are generally characterized by a catechol moiety and their clinical use has therefore been limited to invasive therapies. Achieving sufficient selectivity has also been challenging due to the high degree of homology in the ligand binding site between dopamine receptors subtypes (e.g. dopamine D1 and D5). Also, D1 agonists are associated with potentially limiting side effects including but not limited to dyskinesia and hypotension.

There is therefore a need to design new agents that could modulate D1 receptors.

There has been much interest in the identification of allosteric modulators of GPCRs, both as tools to understand receptor mechanisms and as potential therapeutic agents. GPCRs represent the largest family of cell-surface receptors and a large number of marketed drugs directly activate or block signaling pathways mediated by these receptors. However, for some GPCRs (e.g. peptide receptors), it has proven challenging to develop small molecules or to achieve sufficient selectivity due to the high degree of homology in the ligand binding site between subtypes (e.g. dopamine D1 and D5 or D2 and D3). Accordingly, much drug research has shifted to the identification of small molecules which target sites distinct from the orthosteric natural agonist. Ligands which bind to these sites induce a conformational change in the GPCR thereby allosterically modulating the receptor function. Allosteric ligands have a diverse range of activities including the ability to potentiate (positive allosteric modulator, PAM) or attenuate (negative allosteric modulator, NAM) the effects of the endogenous ligand, by affecting affinity and/or efficacy. As well as subtype selectivity, allosteric modulators may present other potential advantages from a drug discovery perspective such as a lack of direct effect or intrinsic efficacy; only potentiating the effect of the native transmitter where and when it is released; reduced propensity for inducing desensitization arising from constant exposure to an agonist as well as reduced propensity to induce target-related side-effects.

The compounds according to the present invention potentiates the effect of D1 agonists or of the endogenous ligand on D1 receptors through an allosteric mechanism and is therefore a D1 Positive Allosteric Modulator (D1 PAM).

The compounds in accordance with the present invention, being D1 PAMs, are therefore beneficial in the treatment and/or prevention of diseases and disorders in which D1 receptors play a role. Such diseases include cognitive and negative symptoms in schizophrenia, cognitive impairment related to neuroleptic therapy, Mild cognitive impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction, sleep disorders, apathy, traumatic spinal cord injury or neuropathic pain.

International patent application WO 2017/178377 discloses certain substituted 3,4-dihydroisoquinol-2(1H)-yl derivatives and analogs thereof useful as D1 postive allosteric modulators.

International patent application WO2019/204418 discloses certain pyrazo-tetrahydroisoquinolines derivatives which are D1 positive allosteric modulators and may be useful in the treatment of Parkinson's disease, Alzheimer's disease, schizophrenia, and Attention-Deficit Hyperactivity Disorder (ADHD).

However, there remains a need to develop potent D1 positive allosteric modulators combining advantageous pharmacokinetic and pharmacodynamic properties while reducing side effects traditionally associated with treatments involving selective D1 agonists, such as for example hypotension or dyskinesia.

The subject-matter for which protection is sought is as defined by the claims. Any reference to a "disclosure" or an "embodiment" not falling within the scope of the claims is present for explanatory purposes only and does not form part of the invention.

The present invention provides a compound of formula (I), or a pharmaceutically acceptable salt thereof, 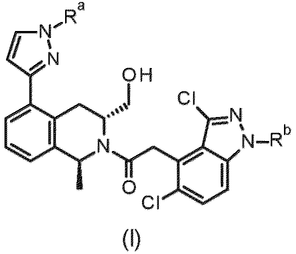 wherein R^{a} and R^{b} represent independently hydrogen or C₁₋₆ alkyl.

The term " C₁₋₆ alkyl" as used herein refers to aliphatic hydrocarbon groups which may be straight or branched and may comprise 1 to 6 carbon atoms in the chain. Suitable alkyl groups which may be present on the compounds of use in the invention include straight-chained and branched C₁₋₄ alkyl groups. Illustrative C₁₋₆ alkyl groups include methyl, ethyl, propyl and butyl.

Formula (I) and the formulae depicted hereinafter are intended to represent all individual stereoisomers and all possible mixtures thereof, unless stated or shown otherwise.

Stereoisomers of compounds of formula (I) include cis and trans isomers, optical isomers such as R and S enantiomers, diastereomers, geometric isomers, rotational isomers, atropisomers, and conformational isomers of the compounds of formula (I), including compounds exhibiting more than one type of isomerism; and mixtures thereof (such as racemates and diastereomeric pairs).

Compounds of formula (I) include asymmetric carbon atoms. The carbon-carbon bonds of the compounds of formula (I) are depicted herein using a solid line ( 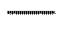 ), a solid wedge ( 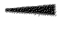 ), or a dotted wedge ( 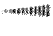 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that only the stereoisomer shown is meant to be included. It is possible that compounds of formula (I) may contain more than one asymmetric carbon atom. In those compounds, the use of a solid line to depict bonds to asymmetric carbon atoms is meant to indicate that all possible stereoisomers are meant to be included.

Some of the compounds of formula (I) may exist in tautomeric forms. Such forms although not explicity indicated in the above formula are intended to be included within the scope of the present invention. Examples of tautomers include keto (CH₂C=O)↔enol (CH=CHOH) tautomers or amide (NHC=O)↔hydroxyimine (N=COH) tautomers. Formula (I) and the formulae depicted hereinafter are intended to represent all individual tautomers and all possible mixtures thereof, unless stated or shown otherwise.

It is also to be understood that each individual atom present in formula (I), or in the formula depicted hereinafter, may in fact be present in the form of any of its naturally occurring isotopes, with the most abundant isotope(s) being preferred. Thus, by way of example, each individual hydrogen atom present in formula (I), or in the formula depicted hereinafter, may be present as a ¹H, ²H (deuterium) or ³H (tritium) atom, preferably ¹H or ²H. Similarly, by way of example, each individual carbon atom present in formula (I), or in the formulae depicted hereinafter, may be present as a ¹²C, ¹³C or ¹⁴C atom, preferably ¹²C.

Specific embodiments of compounds of formula (I) according to the present invention are described hereafter.

In one embodiment according to the present invention, R^{a} represents hydrogen. In another embodiment according to the present invention R^{a} represents C₁₋₆ alkyl. In a particular aspect according to this embodiment, R^{a} represents methyl.

In one embodiment according to the present invention, R^{b} represents hydrogen. In another embodiment according to the present invention R^{b} represents C₁₋₆ alkyl. In a particular aspect according to this embodiment, R^{b} represents methyl.

Therefore, in a particular aspect, the present invention relates to compounds of formula (I) as described in the accompanying Examples.

Illustratively, the present invention relates to compound of formula (I) selected from the group consisting of:
2-(3,5-dichloro-1-methyl-indazol-4-yl)-1-[(1S,3R)-3-(hydroxymethyl)-1-methyl-5-(1-methylpyrazol-4-yl)-3,4-dihydro-1H-isoquinolin-2-yl]ethanone;
2-(3,5-dichloro-1-methyl-indazol-4-yl)-1-[(1S,3R)-3-(hydroxymethyl)-1-methyl-5-(1H-pyrazol-4-yl)-3,4-dihydro-1H-isoquinolin-2-yl]ethanone;
2-(3,5-dichloro-1H-indazol-4-yl)-1-[(1S,3R)-3-(hydroxymethyl)-1-methyl-5-(1H-pyrazol-4-yl)-3,4-dihydro-1H-isoquinolin-2-yl]ethanone; and
2-(3,5-dichloro-1H-indazol-4-yl)-1-[(1S,3R)-3-(hydroxymethyl)-1-methyl-5-(1-methylpyrazol-4-yl)-3,4-dihydro-1H-isoquinolin-2-yl]ethanone.

The present invention also provides a compound of formula (I) as defined above or a pharmaceutically acceptable salt thereof, for use in therapy.

In another aspect, the present invention also provides a compound of formula (I) as defined above, or a pharmaceutically acceptable salt thereof, for use in the treatment of diseases and/or disorders in which D1 receptors play a role.

In another aspect, the present invention provides a compound of formula (I) as defined above, or a pharmaceutically acceptable salt thereof, for use in the treatment and/or prevention of cognitive and negative symptoms in schizophrenia, cognitive impairment related to neuroleptic therapy, Mild Cognitive impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction, sleep disorders, apathy, traumatic spinal cord injury or neuropathic pain.

In a particular embodiment of this aspect, the present invention provides a compound of formula (I) as defined above, or a pharmaceutically acceptable salt thereof for use in the treatment of Parkinson's disease and other movement disorders, Alzheimer's disease, or cognitive and negative symptoms in schizophrenia.

Therefore, in one particular aspect, the present invention provides a compound of formula (I), as defined above, or a pharmaceutically acceptable salt thereof, for use in the treatment of Parkinson's disease and other movement disorders.

In a further aspect, the present invention provides for the use of a compound of formula (I) as defined above, or a pharmaceutically acceptable salt thereof, for the manufacture of a medicament useful for the treatment and/or prevention of diseases and/or disorders in which D1 receptors play a role.

In another further aspect, the present invention provides for the use of a compound of formula (I) as defined above, or a pharmaceutically acceptable salt thereof, for the manufacture of a medicament useful for the treatment and/or prevention of cognitive and negative symptoms in schizophrenia, cognitive impairment related to neuroleptic therapy, Mild Cognitive Impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction, sleep disorders, apathy, traumatic spinal cord injury or neuropathic pain.

In a particular embodiment of this aspect, the present invention provides for the use of a compound of formula (I) as defined above, or a pharmaceutically acceptable salt thereof for the manufacture of a medicament useful for the treatment of Parkinson's disease and other movement disorders, Alzheimer's disease, or cognitive and negative symptoms in schizophrenia.

In one particular aspect, the present invention provides for the use of a compound of formula (I), as defined above, or a pharmaceutically acceptable salt thereof, for the manufacture of a medicament useful for the treatment of Parkinson's disease and other movement disorders.

Also disclosed is a method for the treatment and/or prevention of disorders for which the administration of D1 positive allosteric modulator is indicated, which comprises administering to a patient in need of such treatment an effective amount of a compound of formula (I) as defined above, or a pharmaceutically acceptable salt thereof.

Also disclosed is a method for the treatment and/or prevention of cognitive and negative symptoms in schizophrenia, cognitive impairment related to neuroleptic therapy, Mild Cognitive Impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction, sleep disorders, apathy, traumatic spinal cord injury or neuropathic pain, which comprises administering to a patient in need of such treatment an effective amount of a compound of formula (I) as defined above, or a pharmaceutically acceptable salt thereof.

Also disclosed is a method for the treatment of Parkinson's disease and other movement disorders, Alzheimer's disease, or cognitive and negative symptoms in schizophrenia, which comprises administering to a patient in need of such treatment of an effective amount of a compound of formula (I) as defined above, or a pharmaceutically acceptable salt thereof.

Also disclosed is a method for the treatment of Parkinson's disease and other movement disorders, which comprises administering to a patient in need of such treatment of an effective amount of a compound of formula (I) as defined above, or a pharmaceutically acceptable salt thereof.

Activity in any of the above-mentioned therapeutic indications or disorders can of course be determined by carrying out suitable clinical trials in a manner known to a person skilled in the relevant art for the particular indication and/or in the design of clinical trials in general.

For use in medicine, the salts of the compounds of formula (I) will be pharmaceutically acceptable salts. Other salts may, however, be useful in the preparation of the compounds of use in the invention or of their pharmaceutically acceptable salts. Standard principles underlying the selection and preparation of pharmaceutically acceptable salts are described, for example, in Handbook of Pharmaceutical Salts: Properties, Selection and Use, ed. P.H. Stahl & C.G. Wermuth, Wiley-VCH, 2002. Suitable pharmaceutically acceptable salts of the compound of formula (I) include acid addition salts which may, for example, be formed by mixing a solution of the compound of formula (I) with a solution of a pharmaceutically acceptable acid.

The present invention includes within its scope solvates of the compounds of formula (I) above. Such solvates may be formed with common organic solvents or water.

The present invention also includes within its scope co-crystals of the compounds of formula (I) above. The technical term "co-crystal" is used to describe the situation where neutral molecular components are present within a crystalline compound in a definite stoichiometric ratio. The preparation of pharmaceutical co-crystals enables modifications to be made to the crystalline form of an active pharmaceutical ingredient, which in turn can alter its physicochemical properties without compromising its intended biological activity (see Pharmaceutical Salts and Co-crystals, ed. J. Wouters & L. Quere, RSC Publishing, 2012).

Compounds according to the present invention may exist in different polymorphic forms. Although not explicitly indicated in the above formula, such forms are intended to be included within the scope of the present invention.

Also disclosed are pro-drug forms of the compounds of formula (I) and its various sub-scopes and sub-groups.

For treating diseases, compounds of formula (I) or their pharmaceutically acceptable salts may be employed at an effective daily dosage and administered in the form of a pharmaceutical composition.

Therefore, another embodiment of the present invention concerns a pharmaceutical composition comprising an effective amount of a compound of formula (I) or a pharmaceutically acceptable salt thereof in combination with a pharmaceutically acceptable diluent or carrier.

To prepare a pharmaceutical composition according to the invention, one or more of the compounds of formula (I) or a pharmaceutically acceptable salt thereof is intimately admixed with a pharmaceutical diluent or carrier according to conventional pharmaceutical compounding techniques known to the skilled practitioner.

Suitable diluents and carriers may take a wide variety of forms depending on the desired route of administration, e.g., oral, rectal, parenteral or intranasal.

Pharmaceutical compositions comprising compounds according to the invention can, for example, be administered orally, parenterally, i.e. intravenously, intramuscularly or subcutaneously, intrathecally, by inhalation or intranasally.

Pharmaceutical compositions suitable for oral administration can be solids or liquids and can, for example, be in the form of tablets, pills, dragees, gelatin capsules, solutions, syrups, chewing-gums and the like.

To this end the active ingredient may be mixed with an inert diluent or a non-toxic pharmaceutically acceptable carrier such as starch or lactose. Optionally, these pharmaceutical compositions can also contain a binder such as microcrystalline cellulose, gum tragacanth or gelatine, a disintegrant such as alginic acid, a lubricant such as magnesium stearate, a glidant such as colloidal silicon dioxide, a sweetener such as sucrose or saccharin, or colouring agents or a flavouring agent such as peppermint or methyl salicylate.

The invention also contemplates compositions which can release the active substance in a controlled manner. Pharmaceutical compositions which can be used for parenteral administration are in conventional form such as aqueous or oily solutions or suspensions generally contained in ampoules, disposable syringes, glass or plastics vials or infusion containers.

In addition to the active ingredient, these solutions or suspensions can optionally also contain a sterile diluent such as water for injection, a physiological saline solution, oils, polyethylene glycols, glycerine, propylene glycol or other synthetic solvents, antibacterial agents such as benzyl alcohol, antioxidants such as ascorbic acid or sodium bisulphite, chelating agents such as ethylene diamine-tetra-acetic acid, buffers such as acetates, citrates or phosphates and agents for adjusting the osmolarity, such as sodium chloride or dextrose.

These pharmaceutical forms are prepared using methods which are routinely used by pharmacists.

The amount of active ingredient in the pharmaceutical compositions can fall within a wide range of concentrations and depends on a variety of factors such as the patient's sex, age, weight and medical condition, as well as on the method of administration. Thus, the quantity of compound of formula (I) in compositions for oral administration is at least 0.5 % by weight and can be up to 80 % by weight with respect to the total weight of the composition.

In accordance with the invention it has also been found that the compounds of formula (I) or the pharmaceutically acceptable salts thereof can be administered alone or in combination with other pharmaceutically active ingredients.

In compositions for parenteral administration, the quantity of compound of formula (I) present is at least 0.5 % by weight and can be up to 33 % by weight with respect to the total weight of the composition. For the preferred parenteral compositions, the dosage unit is in the range 0.5 mg to 3000 mg of compounds of formula (I).

The daily dose can fall within a wide range of dosage units of compound of formula (I) and is generally in the range 0.5 to 3000 mg. However, it should be understood that the specific doses can be adapted to particular cases depending on the individual requirements, at the physician's discretion.

It will be apparent to the person skilled in the art that there are various synthetic pathways that can lead to the compounds according to the invention. The following processes are aimed at illustrating some of these synthetic pathways but should not be construed in any way as a limitation on how the compounds according to the invention should be made.

Compound of formula (I) may be prepared by a process involving reacting an intermediate of formula (II) with an intermediate of formula (III), 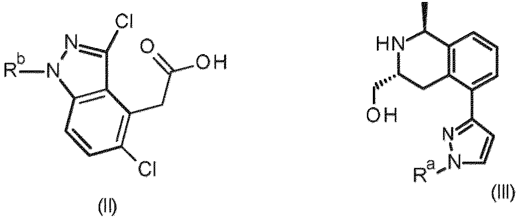 wherein R^{a} and R^{b} are as defined here above.

Intermediate (III) may be conveniently reacted with intermediate of formula (II) in the presence of chloro-N,N,N',N'-tetramethylformamidinium hexafluorophosphate (TCFH) or another coupling agent known to the person skilled in the art, in a suitable solvent, e.g. acetonitrile, with an excess amount of a base, e.g. triethylamine.

Intermediate (III) may be prepared by a process involving reaction of an intermediate of formula (IV), 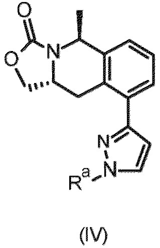 wherein R^{a} is as defined here above.

The reaction is conveniently effected in the presence of a strong base, e.g. sodium hydroxide, in a suitable solvent, e.g. mixture of ethanol and water, a high temperature.

Intermediate of formula (IV) may be prepared by a process involving intermediate (V), 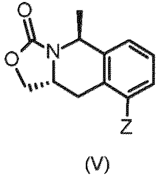 wherein
Z represents a halogen.

The reaction is conviently effected in the presence of tert-butyl 4-(4,4,5,5-tetramethyl-1,3,2-dioxaborolan-2-yl)pyrazole-1-carboxylate or 1-methyl-4-(4,4,5,5-tetramethyl-1,3,2-dioxaborolan-2-yl)-1H-pyrazole, in a suitable solvent, e.g. 1,4-dioxane/water mixture, in the presence of a base, e.g. potassium carbonate, and a suitable catalyst, tetrakis(triphenylphosphine)palladium, at high temperature.

Intermediate of formula (V) may be prepared by a multi-step process from intermediate of formula (VI), 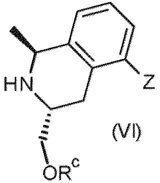
wherein Rc represents tert-butyl-dimethylsilyl; and
Z is as defined above for intermediate (V).
   (i) The amino group of intermediate of formula (VI) is first protected according to conventional methods known to the skilled person in the art or to methods described in the accompanying Examples.
   (ii) Then, both protective groups (R^{c} and protective group on the amino group) are removed by addition of an acid, e.g. HCl, in a suitable solvent, e.g. 2-propanol.
   (iii) Resulting HCl salt of intermediate of formula (VI), wherein Z is as defined above and R^{c} represents hydrogen, thus obtained is reacted with 1,1'-carbonyldiimidazole, in the presence of a suitable base, e.g. N,N-diisopropylethylamine, in a suitable solvent, e.g. dichloromethane, to afford intermediate (V).

Alternatively, compound of formula (I) may be prepared by a multi-step process involving reaction of an intermediate of formula (II) as defined above with an intermediate of formula (VI) as defined above.

The first step is a coupling reaction in the presence of (2-(1H-benzotriazol-1-yl)-1,1,3,3-tetramethyluronium hexafluorophosphate (HBTU) or another coupling agent known to the person skilled in the art, in a suitable solvent, e.g. dimethylformamide, with an excess amount of a base, e.g. N,N-diisopropylethylamine.

The second step, involves another coupling reaction of the intermediate obtained in the first step, with 1-methyl-4-(4,4,5,5-tetramethyl-1,3,2-dioxaborolan-2-yl)-1H-pyrazole in the presence of a suitable catalyst e.g. by tetrakis(triphenylphosphine)palladium(0), in a suitable solvent e.g. dioxane/water mixture, in the presence of a suitable base, e.g. potassium carbonate.

In a third step, the tert-butyl-dimethylsilyl protective group is removed by reaction with cesium fluoride, in a suitable solvent, e.g. dimethyl formamide, to afford compound of formula (I).

Intermediate of formula (VI) may be prepared by a process involving reaction of an intermediate of formula (VII), wherein Z and R^{c} are as defined above for intermediate of formula (V).

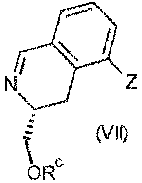

The reaction is conveniently effected in the presence of methyl magnesium chloride, in a suitable solvent e.g. tetrahydrofuran, at low temperature.

Intermediate of formula (VII) may be prepared by a two-steps process involving reaction of intermediate of formula (VIII), 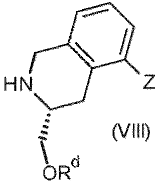 wherein Z is as defined above for intermediate of formula (V) and R^{d} represents hydrogen or tert-butyl-dimethylsilyl.

In a first step intermediate (VIII) wherein R^{d} represents hydrogen is reacted with tert-butyldimethylsilyl chloride in the presence of a suitable base e.g. 4-dimethylamino-pyridine at room temperature, to afford intermediate (VI) wherein R^{d} represents tert-butyl-dimethylsilyl.

In a second step, intermediate (VIII) wherein R^{d} represents tert-butyl-dimethylsilyl is reacted with N-Chlorosuccinimide (NCS), in a suitable solvent, e.g. THF to afford intermediate (VII).

Intermediate (VIII) wherein wherein R^{d} represents hydrogen may be prepared by a process involving intermediate of formula (IX), wherein Z is as defined above for intermediate (V).

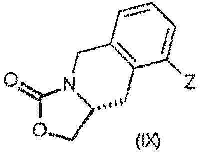

The reaction is conveniently effected in the presence of a strong base, e.g. sodium hydroxide, in a suitable solvent, e.g. mixture of ethanol and water, a high temperature.

Intermediate of formula (IX) may be prepared by a process involving reaction of intermediate (X), 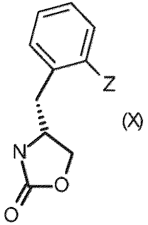 wherein Z is as defined here above for intermediate of formula (V).

The reaction is conveniently effected in the presence of trimethylsilyltriflate and paraformaldehyde, in a suitable solvent e.g. dichloromethane.

Intermediate (X) may be prepared by a two-steps process involving commercially available intermediate (XI), 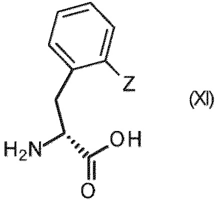 wherein Z is as defined above for intermediate (V).

The reaction is conveniently effected according to the methods described in the accompanying examples or according to methods known to the person skilled in the art.

Intermediate of formula (II), may be prepared by a process involving reacting intermediate of formula (XII), 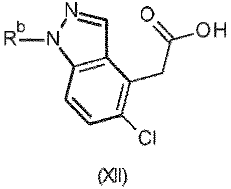 wherein R^{b} is as defined above for compound of formula (I).

The reaction is conveniently effected in the presence of N-chlorosuccinimide, in a suitable solvent, e.g. dimethyl formamide.

Intermediates of formula (XII) may be prepared according to methods described in international patent applications WO 2016/055479 and WO 2017/178377.

Where a mixture of products is obtained from any of the processes described above for the preparation of compounds or intermediates according to the invention, the desired product can be separated therefrom at an appropriate stage by conventional methods such as preparative HPLC; or normal phase column chromatography utilising, for example, silica and/or alumina in conjunction with an appropriate solvent system.

Where the above-described processes for the preparation of the compounds according to the invention give rise to mixtures of stereoisomers, these isomers may be separated by conventional techniques. In particular, where it is desired to obtain a particular enantiomer of a compound of formula (I) or of intermediates (II) or (III) this may be produced from a corresponding mixture of enantiomers using any suitable conventional procedure for resolving enantiomers. Thus, for example, diastereomeric derivatives, e.g. salts, may be produced by reaction of a mixture of enantiomers of formula (I), e.g. a racemate, and an appropriate chiral compound, e.g. a chiral base. The diastereomers may then be separated by any convenient means, for example by crystallisation, and the desired enantiomer recovered, e.g. by treatment with an acid in the instance where the diastereomer is a salt. In another resolution process a racemate of formula (I) may be separated using chiral HPLC or chiral SFC.

Moreover, if desired, a particular enantiomer may be obtained by using an appropriate chiral intermediate in one of the processes described above. Alternatively, a particular enantiomer may be obtained by performing an enantiomer-specific enzymatic biotransformation, e.g. an ester hydrolysis using an esterase, and then purifying only the enantiomerically pure hydrolysed acid from the unreacted ester antipode. Chromatography, recrystallisation and other conventional separation procedures may also be used with intermediates or final products where it is desired to obtain a particular geometric isomer of the invention. Alternatively, the non-desired enantiomer may be racemized into the desired enantiomer, in the presence of an acid or a base, according to methods known to the person skilled in the art, or according to methods described in the accompanying Examples.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 3rd edition, 1999. The protecting groups may be removed at any convenient subsequent stage utilising methods known from the art.

The compounds of formula (I) according to the present invention does not directly activate the dopamine D1 receptor, but potentiates the effect of D1 agonists or the endogenous ligand on D1 receptors, dopamine, through an allosteric mechanism, and is therefore D1 positive allosteric modulator (D1 PAM).

Dopamine and other D1 agonists directly activate the dopamine D1 receptor by themselves.

Assays have been designed to measure the effects of compounds in accordance with the present invention in the absence of dopamine ("activation assay") and in the presence of dopamine ("potentiation assay").

The activation assay measures the stimulation of the production of cyclic adenosinemonophosphate (cAMP) in the Homogeneous Time Resolved Fluorescent (HTRF) assay, with the maximum increase in cAMP by increasing concentrations of the endogenous agonist, dopamine, defined as 100% activation.

When tested, compounds of formula (I) according to the present invention lacks significant direct agonist-like effects in that it produces less than 20% of activation (compared to dopamine maximal response) when present in a concentration of 10 µM.

The potentiation assay measures the ability of compounds to increase the levels of cAMP produced by a low-threshold concentration of dopamine. The concentration of dopamine used ([EC20]) is designed to produce 20% stimulation compared to the maximal response (100%) seen with increasing the concentration of dopamine. To measure this potentiation increasing concentrations of the compound with the [EC20] of dopamine are incubated and the potentiation is measured as increases in cAMP production and concentration of compound which produces 50% of the potentiation of the cAMP levels is measured.

When tested in the cAMP HTRF assay, compound of formula (I) according to the present invention have exhibited values of pEC50 of greater than about 7.5, suitably greater than about 8.0, which shows that they are D1 Positive Allosteric Modulators.

GABA_{A} receptor inhibition is known to be intimately linked to seizures and epilepsy. It is therefore desirable to develop compounds which are D1 Positive Allosteric Modulators and which at the same time minimize such effects.

When tested in a GABA-A receptor inhibition assay as described herein, it is therefore desirable that compound of formula (I) displays a percentage of inhibition of the GABA_{A} receptor of less than about or equal to 10%, when measured at a concentration of 10 µM of a compound of formula (I).

### cAMP HTRF assay

The particular conditions in which the compounds have been tested are described here below.

### a. METHODS D1 Cell culture

Cells were cultured at 37°C in a humidified atmosphere of 5% CO₂. Cells were grown in DMEM-F12+GlutaMAX^{™}-I medium (GIBCO^{®}, Invitrogen, Merelbeke, Belgium) containing 10% fetal bovine serum (BioWhittaker^{®}, Lonza, Verviers, Belgium), 400 µg/mL Geneticin (GIBCO^{®}), 100 IU/mL Penicillin and 100 IU/mL Streptomycin (Pen-Strep solution, BioWhittaker^{®}). LMtk (Ltk-) mouse fibroblast cells expressing the dopamine D1 receptor (BioSignal Inc, Montreal, Canada, now Perkin Elmer) were used as they have been shown to couple efficiently and give robust functional responses (Watts *et al,* 1995).

### b. cAMP assay

The measurement of changes in intracellular cyclic adenosinemonophopshpate (cAMP) was determined using the HTRF cAMP dynamic assay kit from CisBio (Codolet, France). Using homogenous time-resolved fluoresence technology, the assay is based on competition between native cAMP produced by cells and cAMP labelled with the dye d2. The tracer binding is determined by an anti-cAMP antibody labeled with cryptate. The effects of the compound alone (agonism) was determined by performing the assay in the absence of dopamine, whilst the effect of the compound as a positive allosteric modulator (PAM) was determined in the presence of an EC₂₀ concentration of dopamine. Cells (20,000 per well) are incubated in 384 plates for 1 hour at room temperature in a final volume of 20 µLHBSS (Lonza, with calcium, magnesium and HEPES buffer 20 mM, pH 7.4) containing: isobutyl methylxanthine (Sigma, 0.1 mM final), varying concentrations of test compound (typically 10^{-9.5}M to 10^{-4.5}M) in the presence and absence of dopamine (1.1 nM final). The reaction is then terminated and the cells lysed by adding the d2 detection reagent in lysis buffer (10 microL) and the cryptate reagent in lysis buffer (10 microl) according to manufacturer's instructions. This is then incubated for a further 60 min at room temperature and changes in HTRF fluorescent emission ratio determined according to manufacturer's instructions using an Envision plate reader (Perkin Elmer, Zaventem, Belgium) with laser excitation. All incubations were performed in duplicate and results were compared to a concentration-effect curve to dopamine. (10⁻¹¹M to 10⁻⁶M).

### c. Data analysis

Data was analyzed using Excel and PRISM (GraphPad Software) to obtain pEC₅₀ and Erel using the 4-parameter logistic equation (DeLean et al, 1978) where Erel is the fitted maximal response of the test compound minus basal expressed as a percentage relative to that obtained with dopamine which was defined as 100%.

The pEC₅₀ of a compound is the -log10 of the concentration of the compound which produces 50% of the potentiation of the cAMP levels.

The Erel is the relative efficacy, defined as the maximal % potentiation produced by the compound compared to the maximal response produced by increasing concentrations of dopamine (Erel of 1= dopamine maximum response), has been measured.

When tested in the above assay, compounds of formula (II) according to the Examples exhibit the following pEC50 and Erel values :

| **Example #** | **pEC50** | **Erel (%)** |
|---|---|---|
| 1 | 7.9 | 74 |
| 2 | 8.2 | 71 |
| 3 | 8.2 | 61 |
| 4 | 8.1 | 63 |

### Automated Patch Clamp studies on the GABA_{A} receptor Cells

CHO-K1 cells stably expressing human GABA_{A} receptor α1,β2 and γ2 subunits were used. The cells were harvested using trypsin and maintained in serum-free medium at room temperature. The cells were washed and re-suspended in extracellular solution before testing.

### Patch clamp studies

Experiments on human GABA_{A} (α₁β₂γ₂) channels were conducted using an automated patch clamp assay (IonFlux^{™} HT). Compounds were tested at 3 concentrations (0.1, 1, and 10µM) in 3 to 4 cells. The external solution for recording GABA_{A} currents was composed of sodium chloride 137mM, potassium chloride 4 mM, calcium chloride 1.8mM, magnesium chloride 1mM, HEPES 10mM, and glucose 10 mM. Both external and internal solutions were titrated with NaOH or KOH to obtain a pH of 7.35 or 7.3, respectively. The internal pipette solution contained potassium fluoride 70mM, potassium chloride 60mM, sodium chloride 70mM, HEPES 5mM, EGTA 5mM, and Magnesium ATP 4mM. The final concentration of vehicle used to dilute compounds was 0.33% DMSO in each well. Bicuculline (0.032 to 100µM) was used as positive control inhibitor. GABA (15µM) was used as agonist. All recordings were obtained from a holding potential of -60mV.

The compound addition sequence was the following: one addition of the EC₈₀ concentration of GABA was added to establish baseline response. Each concentration of compound was applied for 30 seconds followed by the addition of 15µM) GABA in the presence of the compound for 2 seconds. The process was repeated with the next ascending concentration of compound. Peak inward currents in response to the GABA additions in the presence of a single concentration of compound were measured. All compound data have been normalized to the baseline peak current induced by addition of 15µM) GABA for 2 seconds.

When tested in the above mentioned assay, at a concentration of 10µM, compounds of formula (I) according to the Examples exhibit the following percentage of inhibition of the GABA_{A} :

| **Example #** | **% GABA_{A} inhibition** |
|---|---|
| **1** | 2.7 |
| **2** | 0 |
| **3** | 7.8 |
| **4** | 10.1 |

The following Examples illustrates the preparation of compounds of formula (I) according to the present invention.

### EXAMPLES

The following Examples illustrates the preparation of compounds of formula (I) according to the present invention.

### Abbreviations/recurrent reagents

ACN: Acetonitrile
Brine: Saturated aqueous sodium chloride solution
*n*Bu: *n*-butyl
*t*Bu: *tert*-butyl
COMU: (1-Cyano-2-ethoxy-2-oxoethylidenaminooxy)dimethylamino-morpholino-carbenium hexafluorophosphate
DCM: Dichloromethane
DIPEA: N,N-Diisopropylethylamine
DMAP: 4-Dimethylaminopyridine
DMF: *N,N*-Dimethylformamide
DMSO: Dimethylsulfoxide
EC_{20/50}: concentration which produces 20%/50% of the maximum response
Erel: relative efficacy
ESI: Electrospray Positive lonisation
Et: Ethyl
EtOH : Ethanol
Et₂O: Diethyl ether
EtOAc: Ethyl acetate
h: Hour
HBTU: [Benzotriazol-1-yloxy(dimethylamino)methylene]-dimethyl-ammonium
HPLC: High Pressure Liquid Chromatography
HTRF: homogenous time-resolved fluorescence
LCMS: Liquid Chromatography Mass Spectrometry
MeOH: Methanol
min.: minutes
NCS: *N*-Chlorosuccinimide
NMR: Nuclear magnetic resonance
*i*PrOH: isopropanol
rt: room temperature
SFC: Supercritical Fluid Chromatography
TEA: Triethylamine
THF: Tetrahydrofuran
TLC: Thin Layer Chromatography
cAMP: cyclic adenosinemonophosphate
IUPAC names have been determined using Biovia Draw 20.1.

### Analytical methods

All reactions involving air or moisture-sensitive reagents were performed under a nitrogen or argon atmosphere using dried solvents and glassware. Commercial solvents and reagents were generally used without further purification, including anhydrous solvents when appropriate (generally Sure-Seal^{™} products from Aldrich Chemical Company or AcroSeal^{™} from ACROS Organics). In general reactions were followed by thin layer chromatography, HPLC or mass spectrometry analyses.

Mass spectrometric measurements in LCMS mode are performed using different methods and instrument as follows:

### - Basic LCMS Method 1:

A QDA Waters simple quadrupole mass spectrometer is used for LCMS analysis. This spectrometer is equipped with an ESI source and an UPLC Acquity Classic with diode array detector (210 to 400 nm). Data is acquired in a full MS scan from m/z 70 to 800 in positive/negative modes with a basic elution. The reverse phase separation is carried out at 45 °C on a Waters Acquity UPLC BEH C18 1.7 µm (2.1x50 mm) column for basic elution. Gradient elution is done with H₂O/ACN/ammonium formate (95/5/63 mg/L) + 100 µL/L NH₄OH (solvent A) and ACN/H₂O/ammonium formate (95/5/63 mg/L) + 100 µL/L NH₄OH (solvent B). Injection volume: 1 µL. Full flow in MS.

| **Time (min)** | **A (%)** | **B (%)** | **Flow (mL/min)** |
|---|---|---|---|
| 0 | 99 | 1 | 0.8 |
| 0.15 | 99 | 1 | 0.8 |
| 1.6 | 5 | 95 | 0.8 |
| 1.65 | 5 | 95 | 0.8 |
| 2 | 5 | 95 | 0.8 |
| 2.05 | 99 | 1 | 0.8 |
| 2.75 | 99 | 1 | 0.8 |

### - Basic LCMS Method 2:

A QDA Waters simple quadrupole mass spectrometer is used for LCMS analysis. This spectrometer is equipped with an ESI source and an UPLC Acquity Classic with diode array detector (210 to 400 nm). Data is acquired in a full MS scan from m/z 70 to 800 in positive/negative modes with a basic elution. The reverse phase separation is carried out at 45 °C on a Waters Acquity UPLC BEH C18 1.7 µm (2.1x50 mm) column for basic elution. Gradient elution is done with H₂O/ACN/ammonium formate (95/5/63 mg/L) + 100 µL/L NH₄OH (solvent A) and ACN/H₂O/ammonium formate (95/5/63 mg/L) + 100 µL/L NH₄OH (solvent B). Injection volume: 1 µL. Full flow in MS.

| **Time (min)** | **A (%)** | **B (%)** | **Flow (mL/min)** |
|---|---|---|---|
| 0 | 99 | 1 | 0.4 |
| 0.3 | 99 | 1 | 0.4 |
| 3.2 | 0 | 100 | 0.4 |
| 3.25 | 0 | 100 | 0.5 |
| 4 | 0 | 100 | 0.5 |

### - Acid LCMS Method 1:

A QDA Waters simple quadrupole mass spectrometer is used for LCMS analysis. This spectrometer is equipped with an ESI source and an UPLC Acquity with diode array detector (200 to 400 nm). Data is acquired in a full MS scan from m/z 70 to 800 in positive/negative modes with an acidic elution. The reverse phase separation is carried out at 45 °C on a Waters Acquity UPLC HSS T3 1.8µm (2.1x50 mm) column for acidic elution. Gradient elution is done with H₂O/ACN/TFA (95/5/0.05%) (solvent A) and ACN (solvent B).

| **Time (min)** | **A (%)** | **B (%)** | **Flow (mL/min)** |
|---|---|---|---|
| 0 | 99 | 1 | 0.4 |
| 0.3 | 99 | 1 | 0.4 |
| 3.2 | 5 | 95 | 0.4 |
| 3.25 | 5 | 95 | 0.5 |
| 4 | 5 | 95 | 0.5 |

Some reaction mixtures could be treated using Isolute^{®} separator phase cartridges (from Biotage), acidic columns or catch and release SPE (Solid Phase Extraction) cartridges. Crude materials could be purified by normal phase chromatography, preparative TLC, (acidic or basic) reverse phase chromatography, chiral separation, tritiration or recrystallization.

Normal phase chromatography was performed using silica gel columns (100:200 mesh silica gel or cartridges for normal phase column chromatography systems such as Isolera^{™} Four from Biotage^{®} or Teledyne Isco CombiNormal phase column^{®}).

Preparative reverse phase chromatographies are performed as follows:

### - Basic LCMS prep:

LCMS purification (Basic mode, LCMS prep) using SQD Waters single quadrupole mass spectrometer is used for LCMS purification. This spectrometer is equipped with an ESI source, Waters 2525 binary pump coupled with 2767 sample Manager and with diode array detector (210 to 400 nm.) Data are acquired in a full MS scan from m/z 100 to 850 in positive and negative modes with a basic elution.

*LC parameters:* The reverse phase separation is carried out at room temperature on a Waters XBridge OBD MS C18 column (5µm, 30 x 50 mm). Gradient elution is performed with solvent A1 (H₂O + NH₄HCO₃ 10mM + 50µl/L NH₄OH) and solvent B1 (100% ACN) (pH ~8.5). HPLC flow rate: 35 mL/min to 45 mL/min, injection volume: 990 µL. The splitting ratio is set at +/-1/6000 to MS.

| **Time (min)** | **A1 (%)** | **B1(%)** | **Flow (mL/min)** |
|---|---|---|---|
| 0 | 95 | 5 | 35 |
| 1 | 95 | 5 | 35 |
| 7 | 10 | 90 | 35 |
| 7.5 | 5 | 95 | 35 |
| 9 | 5 | 95 | 35 |
| 9.1 | 5 | 95 | 45 |
| 12 | 5 | 95 | 45 |

Products were generally dried under vacuum before final analyses and submission to biological testing.

NMR spectra were recorded on a BRUKER AVANCEIII 400 MHz-Ultrashield NMR Spectrometer fitted with a Windows 7 Professional workstation running Topspin 3.2 software and a 5 mm Double Resonance Broadband Probe (PABBI ¹H/¹⁹F-BB Z-GRD Z82021/0075) or a 1 mm Triple Resonance Probe (PATXI ¹H/ D-¹³C/¹⁵N Z-GRD Z868301/004).

Chemical shifts are referenced to signals deriving from residual protons of the deuterated solvents (DMSO-*d₆*, MeOH-*d₄* or CDCl₃). Chemical shifts are given in parts per million (ppm) and coupling constants (J) in Hertz (Hz). Spin multiplicities are given as broad (br), singlet (s), doublet (d), triplet (t), quartet (q) and multiplet (m).

All final products were analysed by LCMS in both basic and acid modes, as follows:

### - Basic LCMS Method 3:

A QDA Waters simple quadrupole mass spectrometer is used for LCMS analysis. This spectrometer is equipped with an ESI source and an UPLC Acquity Classic with diode array detector (210 to 400 nm). Data is acquired in a full MS scan from m/z 70 to 800 in positive/negative modes with a basic elution. The reverse phase separation is carried out at 45 °C on a Waters Acquity UPLC BEH C18 1.7 µm (2.1x100 mm) column for basic elution. Gradient elution is done with H₂O/ACN/ammonium formate (95/5/63 mg/L) + 100 µL/L NH₄OH (solvent A) and ACN/H₂O/ammonium formate (95/5/63 mg/L) + 100 µL/L NH₄OH (solvent B). Injection volume: 1 µL. Full flow in MS.

| **Time (min)** | **A (%)** | **B (%)** | **Flow (mL/min)** |
|---|---|---|---|
| 0 | 99 | 1 | 0.4 |
| 0.8 | 99 | 1 | 0.4 |
| 5.30 | 0 | 100 | 0.4 |
| 5.35 | 0 | 100 | 0.5 |
| 7.30 | 0 | 100 | 0.5 |
| 7.35 | 99 | 1 | 0.4 |
| 9 | 90 | 1 | 0.4 |

### - Acid LCMS Method 2:

A QDA Waters simple quadrupole mass spectrometer is used for LCMS analysis. This spectrometer is equipped with an ESI source and an UPLC Acquity Hclass with diode array detector (210 to 400 nm). Data are acquired in a full MS scan from m/z 70 to 800 in positive/negative modes with an acidic elution. The reverse phase separation is carried out at 45 °C on a Waters Acquity UPLC HSS T3 1.8 µm (2.1x100 mm) column for acidic elution. Gradient elution is done with H₂O/ACN/TFA (95/5/0.05%) (solvent A) and ACN (solvent B).

| **Time (min)** | **A (%)** | **B (%)** | **Flow (mL/min)** |
|---|---|---|---|
| 0 | 99 | 1 | 0.4 |
| 0.8 | 99 | 1 | 0.4 |
| 5.3 | 5 | 95 | 0.4 |
| 5.35 | 5 | 95 | 0.5 |
| 7.3 | 5 | 95 | 0.5 |
| 7.35 | 99 | 1 | 0.4 |
| 9 | 99 | 1 | 0.4 |

### 1. Preparation of intermediate IIa- 2-13,5-dichloro-1H-indazol-4-yl)acetic acid

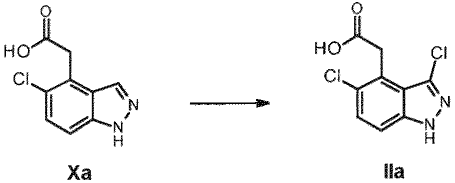

To a solution of 2-(5-chloro-1H-indazol-4-yl)acetic acid **Xa** (CAS: 1904662-08-3, WO2016055479, 2.1 g, 10 mmol) in DMF (10 mL) is added portionwise NCS (1.5g, 11 mmol) at room temperature and the mixture is stirred overnight. The reaction mixture is quenched by adding 100 mL of water dropwise. The product precipitate after stirring during 1h. The solid is filtered and washed twice with the mother liquor phase and twice with water (50 mL). The solid is then dried under vacuum at 45°C overnight to give 2-(3,5-dichloro-1H-indazol-4-yl)acetic acid **IIa** (2.0g, 93% purity, 77% yield), which is used in the next step without further purification.
Acid LCMS Method 1 (ES⁺): 245/247/249 (M+H)⁺
¹H NMR (400 MHz, DMSO-*d₆*): δ 13.52 (s, 1H), 7.52 (d, J = 8.9 Hz, 1H), 7.47 (d, J = 8.9 Hz, 1H), 4.21 (s, 2H)

### 2. Preparation of intermediate lib - 2-(3,5-dichloro-1-methyl-indazol-4-yl)acetic acid

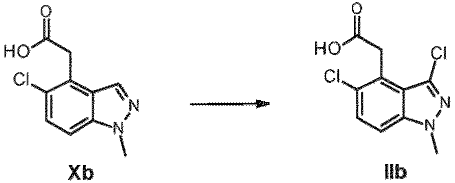

2-(5-Chloro-1-methyl-indazol-4-yl)acetic acid **Xb** (CAS: 2139360-05-5, WO2017178377, 1.3 kg, 5.79 mol) and DMF (6.50 L) are charged into a 50 L three-neck round bottom flask at 20°C. N-Chlorosuccinimide (772 g, 5.79 mol) is added portionwise at 20°C and the mixture is stirred at 20°C for 2h. The reaction mixture is poured into water (25 L) and filtered. The crude product is triturated with isopropyl ether:ethyl acetate (3:1) (7.0 L) at 20°C for 2h then filtered and dried. This overall procedure is carried out on 3 batches of the same size in parallel. The solids obtained from the three batches are combined to give 2-(3,5-dichloro-1-methyl-indazol-4-yl)acetic acid **IIb** (2.1 kg, 7.90 mol, 97.5% purity, 45.5% yield).
¹H NMR (400 MHz, CDCl₃) δ 12.67 (s, 1 H), 7.68 (d, *J* = 9.05 Hz, 1 H), 7.53 (d, *J* = 9.05 Hz, 1 H), 4.20 (s, 2 H), 4.02 (s, 3 H).

### 3. Preparation of intermediate (VI) - [(1S,3R)-5-bromo-1-methyl-1,2,3,4-tertahydroisoquinolin-3-yl]methoxy-tert-butyl-dimethyl-silane

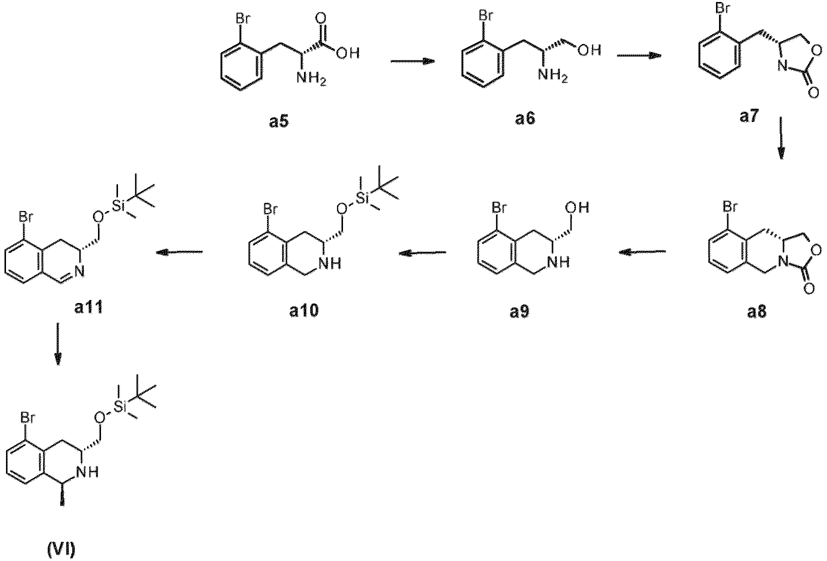

### 3.1. Preparation of intermediate a6 - (2R)-2-amino-3-(2-bromophenyl)propan-1-ol

(2R)-2-amino-3-(2-bromophenyl)propanoic acid as (34.0 kg, 139 mol) and THF (238 L) are charged into a reactor. Sodium borohydride (15.6 kg, 413 mol) is added slowly at 20-30°C. A solution of iodine (35.3 kg, 139 mol) in dry THF (20.0 L) is added slowly at 0-10°C and the reaction mixture is stirred at 70°C for 12h. The reaction is quenched with methanol (70.0 L) at 0°C and heated to 80°C for 30 min. The mixture is cooled down, concentrated under vacuum and the residue is suspended in NaOH (30.0 L, 2N), then filtered. The filter cake is dried under vacuum to give (2R)-2-amino-3-(2-bromophenyl)propan-1-ol a6 as a white solid (31.0 kg, 135 mol, 96.7% yield), which is used in the next step without further purification. ¹H NMR (400 MHz, CDCl₃) δ 7.57 (d, *J* = 7.7 Hz, 1H), 7.21 - 7.29 (m, 2H), 7.07 - 7.15 (m, 1H), 3.66 (dd, *J* = 10.5, 3.6 Hz, 1H), 3.41 (dd, *J* = 10.5, 7.2 Hz, 1H), 3.18 - 3.29 (m, 1H), 2.95 (dd, *J* = 13.5, 5.5 Hz, 1H), 2.70 (dd, *J* = 13.5, 8.2 Hz, 1H), 1.51 - 1.91 (m, 3H).

### 3.2. Preparation of intermediate a7 - (4R)-4-[(2-bromophenyl)methyl]oxazolidin-2-one

(2R)-2-amino-3-(2-bromophenyl)propan-1-ol **a6** (31.0 kg, 135 mol) and dichloromethane (220 L) are charged into a reactor. Triphosgene (13.9 kg, 47.1 mol) is added at room temperature then N,N-diisopropylethylamine (39.1 kg, 303 mol) is slowly added at 0-10°C. The reaction mixture is stirred at 0-10°C for 1h then washed with water (50.0 L) twice, dried with anhydrous sodium sulfate and filtered to give (4R)-4-[(2-bromophenyl)methyl]oxazolidin-2-one **a7** as a solution in dichloromethane which is used directly in the next step.

### 3.3. Preparation of intermediate as - (10aR)-9-bromo-1,5,10,10a-tetrahydrooxazolo[3,4-b]isoquinolin-3-one

A solution of (4R)-4-[(2-bromophenyl)methyl]oxazolidin-2-one **a7** (135 mol) in dichloromethane (220 L) is charged into a reactor and cooled down to 0-5°C. Trimethylsilyl triflate (35.9 kg, 162 mol) and paraformaldehyde (13.3 kg, 148 mol) are added at 0-5°C, then stirred for 2h at 15-20°C. Water (170 L) is added into the mixture which is then extracted twice with dichloromethane (50.0 L). the organic layer is dried with anhydrous sodium sulfate, filtered and concentrated under vacuum. A mixture of petroleum ether:ethyl acetate (1:1, 45.0 L) is added and the mixture is stirred at room temperature for 6h and filtered. The solid is dried to get (10aR)-9-bromo-1,5,10,10a-tetrahydrooxazolo[3,4-b]isoquinolin-3-one **as** as an off-white solid (29.0 kg, 80.2% yield).

¹H NMR (400 MHz, CDCl₃) δ 7.45 - 7.52 (m, 1H), 7.08 - 7.14 (m, 2H), 4.83 (d, *J* = 17.0 Hz, 1H), 4.62 (t, *J* = 8.4 Hz, 1H), 4.36 (d, *J* = 17.0 Hz, 1H), 4.21 (dd, *J* = 8.6, 4.9 Hz, 1H), 3.91 - 3.99 (m, 1H), 3.25 (dd, *J* = 16.3, 4.2 Hz, 1H), 2.67 (dd, *J* = 16.1, 11.0 Hz, 1H).

### 3.4. Preparation of intermediate a9 - [(3R)-5-bromo-1,2,3,4-tetrahydroisoquinolin-3-yl]methanol

Ethanol (120 L) and water (60.0 L) are mixed into a reactor. (10aR)-9-bromo-1,5,10,10a-tetrahydrooxazolo[3,4-b]isoquinolin-3-one **as** (29.7 kg, 111 mol) is added then sodium hydroxide (13.3 kg, 332 mol) is slowly added at 15-20°C. The reaction mixture is stirred at 90°C for 2h then cooled down to room temperature. Water (300 L) is added into the mixture which is centrifugated. The centrifugal cake is dried in circulation oven to give [(3R)-5-bromo-1,2,3,4-tetrahydroisoquinolin-3-yl]methanol **a9** as a white solid (23.7 kg, 88.3% yield) which is used in the next step without further purification.

¹H NMR (400 MHz, CDCl₃) δ 7.37 - 7.47 (m, 1H), 6.95 - 7.08 (m, 2H), 4.00 - 4.10 (m, 2H), 3.85 (dd, *J* = 10.9, 3.7 Hz, 1H), 3.57 (dd, *J* = 10.9, 7.9 Hz, 1H), 3.06 (ddt, *J* = 11.3, 7.6, 4.1, 4.1 Hz, 1H), 2.79 (dd, *J* = 17.1, 4.4 Hz, 1H), 2.40 (dd, *J* = 17.1, 10.9 Hz, 1H), 1.93 (br s, 2H).

### 3.5. Preparation of intermediate a10 - [(3R)-5-bromo-1,2,3,4-tetrahydroisoquinolin-3-yl]methoxy-tert-butyl-dimethyl-silane a10

[(3R)-5-bromo-1,2,3,4-tetrahydroisoquinolin-3-yl]methanol **a9** (23.7 kg, 97.8 mol) and dichloromethane (240 L) are charged into a reactor. DMAP (120 g, 0.98 mol) and imidazole (13.3 kg, 196 mol) are added. Tert-butyldimethylsilyl chloride (TBSCI) (17.7 kg, 117 mol) is slowly added at 15-20°C and the mixture is stirred for 12h. Ammonium chloride (100 L) is added into the mixture. The organic phase is separated, washed with water (50.0 L), dried with anhydrous sodium sulfate, filtered and concentrated under vacuum to give [(3R)-5-bromo-1,2,3,4-tetrahydroisoquinolin-3-yl]methoxy-tert-butyl-dimethyl-silane **a10** as a yellow oil (37.6 kg, 86% purity, 93% yield) which is used in the next step without further purification.

¹H NMR (400 MHz, CDCl₃) δ 7.36 - 7.45 (m, 1H), 7.01 (d, *J* = 4.6 Hz, 2H), 4.01 - 4.13 (m, 1H), 3.84 (dd, *J* = 9.9, 3.7 Hz, 1H), 3.64 (dd, *J* = 9.8, 7.2 Hz, 1H), 2.96 - 3.08 (m, 1H), 2.75 (dd, *J* = 17.0, 4.2 Hz, 1H), 2.44 (dd, *J* = 17.0, 10.8 Hz, 1H), 1.76 - 2.20 (m, 2H), 0.89 - 0.97 (m, 9H), 0.08 - 0.14 (m, 6H).

### 3.6. Preparation of intermediate a11 - [(3R)-5-bromo-3,4-dihydroisoquinolin-3-yl]methoxy-tert-butyl-dimethyl-silane

[(3R)-5-bromo-1,2,3,4-tetrahydroisoquinolin-3-yl]methoxy-tert-butyl-dimethyl-silane **a10** (3.42 kg, 8.31 mol) and THF (30.0 L) are charged into a reactor. N-Chlorosuccinimide (NCS) (1.17 kg, 8.73 mol) is slowly added at room temperature and the mixture is stirred at 25°C for 30 min. A solution of KOH (1.52 kg, 27.1 mol) in dry methanol (7.00 L) is slowly added at room temperature and the reaction is stirred at 25°C for 1h. The reaction is quenched with water (10.0 L) and extracted with petroleum ether:ethyl acetate (1:2, 5.00 L). The organic layer is separated, washed with brine (10.0 L), dried with anhydrous sodium sulfate and filtered. This overall procedure is carried out on 10 batches of the same size in parallel and the 10 reaction filtrates are combined and concentrated under vacuum to give [(3R)-5-bromo-3,4-dihydroisoquinolin-3-yl]methoxy-tert-butyl-dimethyl-silane **a11** as a brown oil (28.0 kg, crude) which is used in the next step without further purification.

¹H NMR (400 MHz, CDCl₃) δ 8.24 (d, *J* = 2.6 Hz, 1H), 7.58 (dd, *J* = 7.8, 1.2 Hz, 1H), 7.12 - 7.25 (m, 2H), 4.03 (dd, *J* = 9.5, 4.0 Hz, 1H), 3.67 - 3.77 (m, 2H), 3.07 (dd, *J* = 17.0, 6.2 Hz, 1H), 2.68 (dd, *J* = 17.1, 10.9 Hz, 1H), 0.88 - 0.91 (m, 9H), 0.07 (d, *J* = 1.5 Hz, 6H).

### 3.7. Preparation of intermediate (VI) - [(1S,3R)-5-bromo-1-methyl-1,2,3,4-tetrahydroisoquinolin-3-yl]methoxy-tert-butyl-dimethyl-silane

[(3R)-5-bromo-3,4-dihydroisoquinolin-3-yl]methoxy-tert-butyl-dimethyl-silane **a11** (3.10 kg, 8.75 mol) and THF (20.0 L) are charged into a reactor. The mixture is cooled down to 0°C and methylmagnesium chloride (3M, 11.6 L) is added. The mixture is stirred at 20°C for 12h. The reaction is quenched with a saturated solution of ammonium chloride. The phases are separated and the aqueous layer is extracted twice with petroleum ether:ethyl acetate (3:1, 5.00 L). The combined organic phases are washed with brine (10.0 L), dried over anhydrous sodium sulfate and filtered. This overall procedure is carried out on 9 batches of the same size in parallel and the nine reaction filtrates are combined and concentrated under vacuum. The crude mixture was purified by silica gel chromatography with petroleum ether : ethyl acetate (10:1) to give [(1S,3R)-5-bromo-1-methyl-1,2,3,4-tetrahydroisoquinolin-3-yl]methoxy-tert-butyl-dimethyl-silane **(VI)** as a brown oil (4.60 kg, 99.7% purity, 15.7% yield).

¹H NMR (400 MHz, DMSO-*d*₆) δ 7.41 (dd, *J*=7.7, 0.9 Hz, 1H), 7.12 - 7.18 (m, 1H), 7.03 - 7.11 (m, 1H), 4.12 (q, *J*=6.8 Hz, 1H), 3.62 (d, *J*=5.7 Hz, 2H), 3.07 - 3.17 (m, 1H), 2.67 - 2.76 (m, 1H), 2.26 (dd, *J*=16.9, 10.0 Hz, 1H), 2.12 (br s, 1H), 1.32 (d, *J*=6.8 Hz, 3H), 0.84 - 0.93 (m, 9H), 0.07 (d, *J*=0.9 Hz, 6H).

### 2. Preparation of Examples 1 and 2 - 2-(3,5-dichloro-1-methyl-indazol-4-yl)-1-[(1S,3R)-3-(hydroxymethyl)-1-methyl-5-(1-methylpyrazol-4-yl)-3,4-dihydro-1H-isoquinolin-2-yl]ethenone and 2-(3,5-dichloro-1-methyl-indazol-4-yl)-1-[(1S,3R)-3-(hydroxymethyl)-1-methyl-5-(1H-pyrazol-4-yl)-3,4-dihydro-1H-isoquinolin-2-yl]ethenone

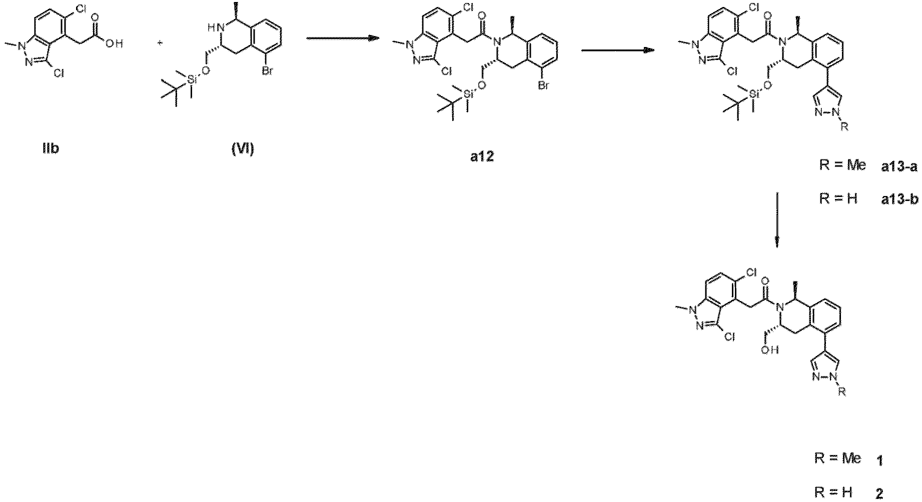

### 2.1. Synthesis of intermediate a12 - 1-[(1S,3R)-5-bromo-3-[[tert-butyl(dimethyl)silyl]oxymethyl]-1-methyl-3,4-dihydro-1H-isoquinolin-2-yl]-2-(3,5-dichloro-1-methyl-indazol-4-yl)ethanone

To a solution of [(1S,3R)-5-bromo-1-methyl-1,2,3,4-tetrahydroisoquinolin-3-yl]methoxy-tert-butyl-dimethyl-silane **(VI)** (500 mg, 1.35 mmol) and 2-(3,5-dichloro-1-methyl-indazol-4-yl)acetic acid **(lib)** (385 mg, 1.48 mmol) in DMF (10 mL) is added HBTU (0.61 g, 1.62 mmol) followed by DIPEA (0.68 mL, 4.05 mmol). The reaction mixture is stirred overnight at rt. Then the reaction mixture is taken up by DCM (50 mL) and washed four times with a saturated aqueous solution of sodium hydrogenocarbonate (4 x 10 mL). The organic layer is dried over MgSO₄, filtered and concentrated under vacuum. The crude residue is purified by normal phase column chromatography (elution: EtOAc/heptane) to afford 1-[(1S,3R)-5-bromo-3-[[tert-butyl(dimethyl)silyl]oxymethyl]-1-methyl-3,4-dihydro-1H-isoquinolin-2-yl]-2-(3,5-dichloro-1-methyl-indazol-4-yl)ethanone **a12** (590 mg, 71% yield) as a white solid.
Basic LCMS Method 2 (ES⁺): 610/612/614 (M+H)⁺

### 2.2. Synthesis of intermediate a13-a - 1-[(1S,3R)-3-[[tert-butyl(dimethyl)silyl]oxymethyl]-1-methyl-5-(1-methylpyrazol-4-yl)-3,4-dihydro-1H-isoquinolin-2-yl]-2-(3,5-dichloro-1-methyl-indazol-4-yl)ethanone

To a solution of 1-[(1S,3R)-5-bromo-3-[[tert-butyl(dimethyl)silyl]oxymethyl]-1-methyl-3,4-dihydro-1H-isoquinolin-2-yl]-2-(3,5-dichloro-1-methyl-indazol-4-yl)ethanone **a12** (0295 mg, 0.48 mmol) and 1-methyl-4-(4,4,5,5-tetramethyl-1,3,2-dioxaborolan-2-yl)-1H-pyrazole (0.15 g, 0.74 mmol) in dioxane/H₂O (7 mL / 0.7 mL) is added potassium carbonate (200 mg, 1.45 mmol) followed by tetrakis(triphenylphosphine)palladium(0) (56 mg, 0.048 mmol). Then the reaction mixture is heated at 115°C for 2h. The reaction mixture is taken up by DCM (50 mL) and washed four times with a saturated aqueous solution of sodium hydrogenocarbonate (4 x 10 mL). The organic layer is dried over MgSO₄, filtered and concentrated under vacuum. The crude residue is purified by normal phase column chromatography (elution: EtOAc/heptane) to afford 1-[(1S,3R)-3-[[tert-butyl(dimethyl)silyl]oxymethyl]-1-methyl-5-(1-methylpyrazol-4-yl)-3,4-dihydro-1H-isoquinolin-2-yl]-2-(3,5-dichloro-1-methyl-indazol-4-yl)ethanone **a13-a** (0.14 g, 49%) as a white solid still containing impurities but used as such in the next step.

Basic LCMS Method 2 (ES⁺): 612/614/616 (M+H)⁺
Same procedure as above using tert-butyl 4-(4,4,5,5-tetramethyl-1,3,2-dioxaborolan-2-yl)pyrazole-1-carboxylate is used to afford 1-[(1S,3R)-3-[[tert-butyl(dimethyl)silyl]oxymethyl]-1-methyl-5-(1H-pyrazol-4-yl)-3,4-dihydro-1H-isoquinolin-2-yl]-2-(3,5-dichloro-1-methyl-indazol-4-yl)ethanone **a13-b** as a white solid (17% yield).

Basic LCMS Method 2 (ES⁺): 598/600/602 (M+H)⁺

### 2.3. Synthesis of Example 1 - 2-(3,5-dichloro-1-methyl-indazol-4-yl)-1-[(1S,3R)-3-(hydroxymethyl)-1-methyl-5-(1-methylpyrazol-4-yl)-3,4-dihydro-1H-isoquinolin-2-yl]ethanone

To a solution of 1-[(1S,3R)-3-[[tert-butyl(dimethyl)silyl]oxymethyl]-1-methyl-5-(1-methylpyrazol-4-yl)-3,4-dihydro-1H-isoquinolin-2-yl]-2-(3,5-dichloro-1-methyl-indazol-4-yl)ethanone **a13-a** (140 mg, 0.230 mmol) in DMF (3 mL), cooled to 0°C, is added cesium fluoride (180 mg, 1.19 mmol). The reaction mixture is heated at 50°C overnight. Then the reaction mixture is taken up by EtOAc (20 mL) and washed three times with a saturated aqueous solution of sodium hydrogenocarbonate (3 x 10 mL). The organic layer is dried over MgSO₄, filtered and concentrated under vacuum. The crude residue is purified by a LCMS purification (Basic LCMS prep) to afford 2-(3,5-dichloro-1-methyl-indazol-4-yl)-1-[(1S,3R)-3-(hydroxymethyl)-1-methyl-5-(1-methylpyrazol-4-yl)-3,4-dihydro-1H-isoquinolin-2-yl]ethanone **1** (30.0 g, 25% yield) as a white solid.
Basic LCMS Method 3 (ES⁺): 498/500/502 (M+H)⁺, 100% purity
Acid LCMS Method 2 (ES⁺): 498/500/502 (M+H)⁺, 100% purity
¹H NMR (400 MHz, CDCl₃) δ 7.61 (d, *J* = 9.3 Hz, 1H), 7.52 (d, *J* = 3.3 Hz, 1H), 7.41 (dd, *J* = 9.0, 4.2 Hz, 1H), 7.31 - 7.19 (m, 3H), 7.14 (dd, *J* = 5.7, 3.1 Hz, 1H), 5.32 - 5.17 (m, 1H), 4.63 - 4.44 (m, 3H), 4.02 - 3.94 (m, 6H), 3.55 - 3.38 (m, 2H), 3.23 - 3.09 (m, 2H), 1.72 (d, *J* = 6.7 Hz, 2H), 1.42 (d, *J* = 6.5 Hz, 1H).

### 2.4. Synthesis of Example 2 - 2-(3,5-dichloro-1-methyl-indazol-4-yl)-1-[(1S,3R)-3-(hydroxymethyl)-1-methyl-5-(1H-pyrazol-4-yl)-3,4-dihydro-1H-isoquinolin-2-yl]ethanone

Same procedure as above is used starting from intermediate **a13-b** to afford 2-(3,5-dichloro-1-methyl-indazol-4-yl)-1-[(1S,3R)-3-(hydroxymethyl)-1-methyl-5-(1H-pyrazol-4-yl)-3,4-dihydro-1H-isoquinolin-2-yl]ethanone **2** (49% yield) as a white solid.
Basic LCMS Method 3 (ES⁺): 484/486/488 (M+H)⁺, 100% purity
Acid LCMS Method 2 (ES⁺): 484/486/488 (M+H)⁺, 100% purity
¹H NMR (400 MHz, CDCl₃) δ 7.69 (s, 1H), 7.63 (s, 1H), 7.40 (dd, *J* = 12.2, 8.9 Hz, 1H), 7.33 - 7.23 (m, 3H), 7.23 - 7.11 (m, 1H), 5.32 - 5.17 (m, 1H), 4.56 (t, *J* = 5.7 Hz, 2H), 4.50 (s, 1H), 3.96 (s, 3H), 3.61 - 3.44 (m, 2H), 3.18 - 3.01 (m, 2H), 1.72 (d, *J* = 6.7 Hz, 2H), 1.41 (d, *J* = 6.5 Hz, 1H).

### 3. Preparation of Examples 3 and 4 - 2-(3,5-dichloro-1H-indazol-4-yl)-1-[(1S,3R)-3-(hydroxymethyl)-1-methyl-5-(1H-pyrazol-4-yl)-3,4-dihydro-1H-isoquinolin-2-yl]ethenone and 2-(3,5-dichloro-1H-indazol-4-yl)-1-[(1S,3R)-3-(hydroxymethyl)-1-methyl-5-(1-methylpyrazol-4-yl)-3,4-dihydro-1H-isoquinolin-2-yl]ethanone

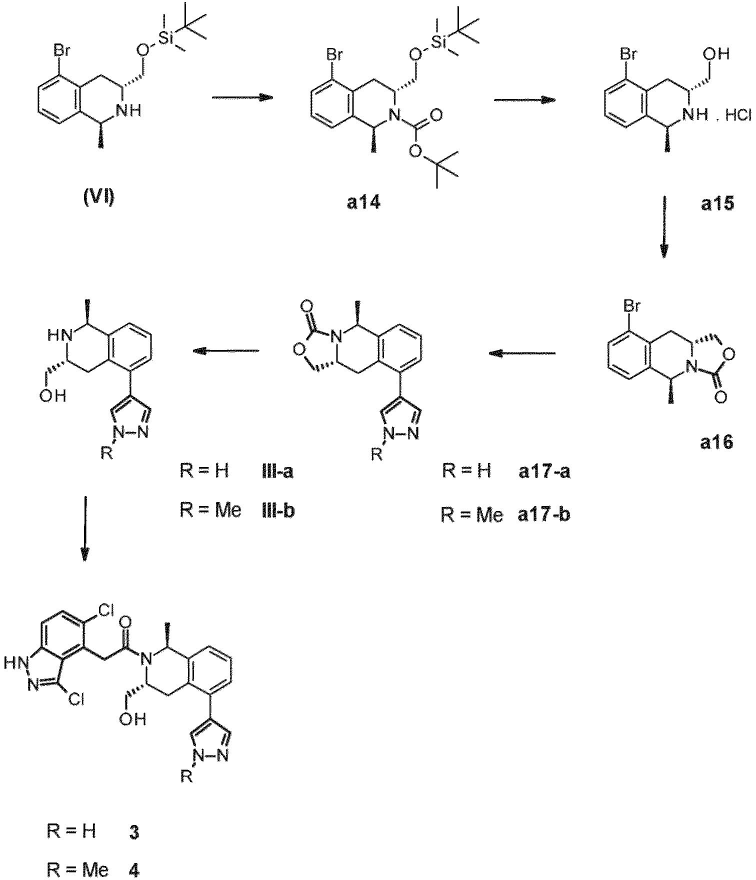

### 3.1. Preparation of intermediate a14 - tert-butyl (1S,3R)-5-bromo-3-[[tert-butyl(dimethyl)silyl]oxymethyl]-1-methyl-3,4-dihydro-1H-isoquinoline-2-carboxylate

[(1S,3R)-5-bromo-1-methyl-1,2,3,4-tetrahydroisoquinolin-3-yl]methoxy-tert-butyl-dimethylsilane **(VI)** (1.85 kg, 4.99 mol) and dichloromethane (13.0 L) are charged in a reactor. N,N-diisopropylethylamine (1.94 kg, 14.9 mol) and di-tert-butyl dicarbonate (1.14 kg, 5.24 mol) are added at room temperature and the mixture is stirred for 12h. The reaction mixture is washed twice with a saturated ammonium chloride solution (10.0 L), the organic layer is dried with anhydrous sodium sulfate and filtered. This overall procedure is carried out on 2 batches of the same size in parallel and the two reaction filtrates are combined and concentrated under vacuum. The crude mixture is purified by silica gel chromatography with petroleum ether : EtOAc (30:1) to give tert-butyl (1S,3R)-5-bromo-3-[[tert-butyl(dimethyl)silyl]oxymethyl]-1-methyl-3,4-dihydro-1H-isoquinoline-2-carboxylate **a14** as a yellow oil (4.00 kg, 99.% purity, 85% yield).

¹H NMR (400 MHz, DMSO-*d*₆) δ 7.50 (d, *J* = 7.9 Hz, 1H), 7.22 (br d, *J* = 6.7 Hz, 1H), 7.06 - 7.18 (m, 1H), 4.84 (br s, 1H), 4.12 (br s, 1H), 3.46 (br d, *J* = 15.4 Hz, 2H), 2.94 (br dd, *J* = 15.8, 5.2 Hz, 1H), 2.71 (br t, *J* = 9.5 Hz, 1H), 1.45 (s, 9 H), 1.28 (br s, 3H), 0.81 (s, 9H), -0.08 (s, 6H).

### 3.2. Preparation of intermediate a15 - [(1S,3R)-5-bromo-1-methyl-1,2,3,4-tetrahydroisoquinolin-3-yl]methanol hydrochloride

To a solution of tert-butyl (1S,3R)-5-bromo-3-[[tert-butyl(dimethyl)silyl]oxymethyl]-1-methyl-3,4-dihydro-1H-isoquinoline-2-carboxylate **a14** (51.0 g, 108 mmol) in 2-propanol (200 mL) is added dropwise at 0°C hydrochloric acid (200 mL, 4M in dioxan) and the resulting mixture is allowed to warm to room temperature overnight. The reaction mixture is evaporated under vacuum and the resulting solid is dried under vacuum for 2h to afford [(1S,3R)-5-bromo-1-methyl-1,2,3,4-tetrahydroisoquinolin-3-yl]methanol hydrochloride **a15** (31.8 g, 99% crude yield), which is used in the next step without further purification.Acid LCMS Method 1 (ES⁺): 256/258 (M+H)⁺

### 3.3. Preparation of intermediate a16 - (5S,10aR)-9-bromo-5-methyl-1,5,10,10a-tetrahydrooxazolo[3,4-b]isoquinolin-3-one

To a solution of [(1S,3R)-5-bromo-1-methyl-1,2,3,4-tetrahydroisoquinolin-3-yl]methanol hydrochloride **a15** (31.8 g, 108 mmol) in DCM (400 mL) is added 1,1'-carbonyldiimidazole (35.0 g, 2156 mmol) at room temperature. The reaction mixture is cooled down to 0°C and stirred for 15 min. N,N-diisopropylethylamine (90 mL, 541.8 mmol) is added dropwise. The mixture is allowed to stir overnight at room temperature. The reaction mixture is then diluted with DCM (200 mL). The resulting organic layer is washed twice with 500 mL of HCl 1M and with 500 mL of water then dried over MgSO₄, filtered and the solvent is removed under vacuum to afford (5S,10aR)-9-bromo-5-methyl-1,5,10,10a-tetrahydrooxazolo[3,4-b]isoquinolin-3-one **a16** (31.8 g, quantitative crude yield), which is used in the next step without further purification.
Acid LCMS Method 1 (ES⁺): 282/284 (M+H)⁺
¹H NMR (DMSO-*d₆*): δ 7.55 (dd, J = 7.9, 1.2 Hz, 1H), 7.36 (d, J = 7.7 Hz, 1H), 7.20 (t, J = 7.8 Hz, 1H), 4.86 (q, J = 6.8 Hz, 1H), 4.55 (t, J = 7.9 Hz, 1H), 4.25 - 4.11 (m, 2H), 3.13 (dd, J = 16.7, 4.5 Hz, 1H), 2.61 (dd, J = 16.7, 10.2 Hz, 1H), 1.43 (d, J = 6.8 Hz, 3H).

### 3.4. Preparation of intermediate a17-a - (5S,10aR)-5-methyl-9-(1H-pyrazol-4-yl)-1,5,10,10a-tetrahydrooxazolo[3,4-b]isoquinolin-3-one

To a mixture of (5S,10aR)-9-bromo-5-methyl-1,5,10,10a-tetrahydrooxazolo[3,4-b]isoquinolin-3-one **a16** (200 mg, 0.71 mmol) and tert-butyl 4-(4,4,5,5-tetramethyl-1,3,2-dioxaborolan-2-yl)pyrazole-1-carboxylate (1.5 eq., 322 mg, 1.06 mmol) in 1,4-dioxane (7 mL) and water (0.7 mL) at rt is sequentially added potassium carbonate (294 mg, 2.13 mmol) and tetrakis(triphenylphosphine)palladium(0) (83.0 mg, 0.070 mmol) and the reaction mixture is heated at 100 °C for 18 h. The reaction mixture is cooled down to rt and EtOAc and a saturated solution of NaHCO₃ are added. The layers are separated, the aqueous layer is extracted with EtOAc twice. Then, the combined organic layers are dried over MgSO₄, filtered and concentrated to dryness to give a yellow oil which is purified by reverse phase flash chromatography Biotage Isolera Four in basic conditions (C18 SNAP 60 g gel column in a gradient from 5% to 70% ACN in Water/NH₄OH over 12 CV). The purest fractions are directly lyophilized to give (5S,10aR)-5-methyl-9-(1H-pyrazol-4-yl)-1,5,10,10a-tetrahydrooxazolo[3,4-b]isoquinolin-3-one **a17-a** (84.0 mg, 44% yield) as a white solid.
Basic LCMS Method 1 (ES⁺): 270 (M+H)⁺
¹H NMR (400 MHz, DMSO-*d*₆) δ 7.81 (s, 2H), 7.29 - 7.19 (m, 3H), 4.86 (q, *J* = 6.7 Hz, 1H), 4.49 (t, *J* = 8.2 Hz, 1H), 4.17 (dd, *J* = 8.6, 5.0 Hz, 1H), 4.09 - 3.97 (m, 1H), 2.98 (dd, *J* = 16.3, 4.5 Hz, 1H), 2.85 (dd, *J* = 16.4, 10.7 Hz, 1H), 1.46 (d, *J* = 6.8 Hz, 3H).

Same procedure as above using 1-methyl-4-(4,4,5,5-tetramethyl-1,3,2-dioxaborolan-2-yl)-1H-pyrazole is used to afford (5S,10aR)-5-methyl-9-(1-methylpyrazol-4-yl)-1,5,10,10a-tetrahydrooxazolo[3,4-b]isoquinolin-3-one **a17-b** (63% yield) as a white solid.
Basic LCMS Method 1 (ES⁺): 284 (M+H)⁺
¹H NMR (400 MHz, DMSO-*d*₆) δ 7.90 (d, J = 0.9 Hz, 1H), 7.77 (s, 0.4H), 7.71 (s, 0.6H), 7.61 (d, J = 0.8 Hz, 1H), 7.30 - 7.19 (m, 2H), 4.86 (q, J = 6.7 Hz, 1H), 4.49 (t, J = 8.2 Hz, 1H), 4.16 (dd, J = 8.6, 4.9 Hz, 1H), 4.09 - 3.97 (m, 1H), 3.88 (s, 3H), 2.99 (dd, J = 16.3, 4.4 Hz, 1H), 2.81 (dd, J = 16.3, 10.8 Hz, 1H), 1.45 (d, J = 6.8 Hz, 3H).

### 3.5. Preparation of intermediates IIIa - [(1S,3R)-1-methyl-5-(1H-pyrazol-4-yl)-1,2,3,4-tetrahydroisoquinolin-3-yl]methanol

To a solution of (5S,10aR)-5-methyl-9-(1H-pyrazol-4-yl)-1,5,10,10a-tetrahydrooxazolo[3,4-b]isoquinolin-3-one **a17-a** (84.0 mg, 0.31 mmol) in ethanol (0.4 mL) at rt is added sodium hydroxide (50% w/w solution in water, 0.08 mL, 1.51 mmol) and the reaction mixture is stirred at 70 °C for 3h30. To the resulting mixture are then added EtOAc and a saturated solution of NaCl and the layers are separated. The aqueous layer is extracted with EtOAc twice. Then, the combined organic layers are dried over MgSO₄, filtered and concentrated to dryness to give a yellow solid yellow oil which is purified by reverse phase flash chromatography Biotage Isolera Four in basic conditions (C18 SNAP 30 g gel column in a gradient from 5% to 95% ACN in Water/NH₄OH over 12 CV). The purest fractions are directly lyophilized to give [(1S,3R)-1-methyl-5-(1H-pyrazol-4-yl)-1,2,3,4-tetrahydroisoquinolin-3-yl]methanol **III-a** (40 mg, 0.14 mmol, 47% yield) as a white solid.

Basic LCMS Method 1 (ES⁺): 244 (M+H)⁺, 87% purity.

Same procedure as above starting from (5S,10aR)-5-methyl-9-(1-methylpyrazol-4-yl)-1,5,10,10a-tetrahydrooxazolo[3,4-b]isoquinolin-3-one **a17-b** is used. The crude mixture is purified by reverse phase preparative HPLC on a Waters XBridge OBD MS C18 column (5µm, 30 x 50 mm), gradient elution is performed with solvent A (H₂O 95% - ACN 5% + NH₄HCO₃ 50 mM + 200µl/L NH₄OH) and solvent B (100% ACN) 95/5 to 5/95 (pH ~8.5) to afford [(1S,3R)-1-methyl-5-(1-methylpyrazol-4-yl)-1,2,3,4-tetrahydroisoquinolin-3-yl]methanol **III-b** (65% yield) as a colorless oil.

Basic LCMS Method 1 (ES⁺): 258 (M+H)⁺, 99% purity.

¹H NMR (400 MHz, DMSO-*d*₆) δ 7.85 (d, J = 0.9 Hz, 1H), 7.57 (d, J = 0.8 Hz, 1H), 7.17 - 7.06 (m, 2H), 7.02 (dd, J = 6.5, 2.7 Hz, 1H), 4.61 (t, J = 5.4 Hz, 1H), 4.16 (q, J = 6.8 Hz, 1H), 3.87 (s, 3H), 3.41 (m, 1H), 3.09 - 2.92 (m, 1H), 2.75 - 2.59 (m, 1H), 2.43 - 2.26 (m, 1H), 1.35 (d, J = 6.8 Hz, 3H).

### 3.6. Preparation of Example 3 - 2-(3,5-dichloro-1H-indazol-4-yl)-1-[(1S,3R)-3-(hydroxymethyl)-1-methyl-5-(1H-pyrazol-4-yl)-3,4-dihydro-1H-isoquinolin-2-yl]ethanone

To a solution of 2-(3,5-dichloro-1H-indazol-4-yl)acetic acid **IIa** (35.0 mg, 0.130 mmol) in acetonitrile (0.5 mL) at 0 °C is sequentially added triethylamine (0.047 mL, 0.33 mmol), [(1S,3R)-1-methyl-5-(1H-pyrazol-4-yl)-1,2,3,4-tetrahydroisoquinolin-3-yl]methanol **a18-a** (40.0 mg, 0.140 mmol) and chloro-N,N,N',N'-tetramethylformamidinium hexafluorophosphate (TCFH, 44.0 mg, 0.150 mmol) and the reaction mixture is allowed to stir at rt for 4 h. To the resulting mixture are then added EtOAc and a saturated solution of NH₄Cl and the layers are separated. The aqueous layer is extracted with EtOAc twice. Then, the combined organic layers are dried over MgSO₄, filtered and concentrated to dryness to give a yellow solid which is purified by reverse phase preparative HPLC on a Waters XBridge OBD MS C18 column (5µm, 30 x 50 mm), gradient elution is performed with solvent A ( H₂O 95% - ACN 5% + NH₄HCO₃ 50 mM + 200µl/L NH₄OH) and solvent B (100% ACN) (pH ~8.5) 25% to 45% to afford 2-(3,5-dichloro-1H-indazol-4-yl)-1-[(1S,3R)-3-(hydroxymethyl)-1-methyl-5-(1H-pyrazol-4-yl)-3,4-dihydro-1H-isoquinolin-2-yl]ethanone 3 (3.60 mg, 6% yield) as a white solid.

Basic LCMS Method 3 (ES⁺): 470/472/474 (M+H)⁺, 99% purity.

Acid LCMS Method 2 (ES⁺): 470/472/474 (M+H)⁺, 100% purity.

¹H NMR (400 MHz, DMSO-*d*₆) δ 12.96 (s, 1H), 7.89 (bs, 3H), 7.48 (d, J = 8.9 Hz, 1H), 7.39 (s, 1H), 7.33 - 7.19 (m, 2H), 7.16 - 7.10 (m, 1H), 5.40 - 5.32 (m, 0.3H), 5.08 (q, J = 6.5 Hz, 0.7H), 4.93 (dd, J = 6.2, 4.0 Hz, 0.7H), 4.72 - 4.32 (m, 3.3H), 3.52 - 3.30 (m, 1H), 3.10 - 2.75 (m, 2H), 1.57 (d, J = 6.6 Hz, 0.9H), 1.35 - 1.21 (m, 2.1H).

### 3.7. Preparation of Example 4 - 2-(3,5-dichloro-1H-indazol-4-yl)-1-[(1S,3R)-3-(hydroxymethyl)-1-methyl-5-(1-methylpyrazol-4-yl)-3,4-dihydro-1H-isoquinolin-2-yl]ethanone

Same procedure as above starting from [(1S,3R)-1-methyl-5-(1-methylpyrazol-4-yl)-1,2,3,4-tetrahydroisoquinolin-3-yl]methanol **a18-b** is used. The obtained crude mixture is purified by reverse phase preparative HPLC on a Waters XBridge OBD MS C18 column (5µm, 30 x 50 mm), gradient elution is performed with solvent A (H₂O 95% - ACN 5% + NH₄HCO₃ 50 mM + 200µl/L NH₄OH) and solvent B (100% ACN) 75/25 to 50/50 (pH ~8.5) to afford 2-(3,5-dichloro-1H-indazol-4-yl)-1-[(1S,3R)-3-(hydroxymethyl)-1-methyl-5-(1-methylpyrazol-4-yl)-3,4-dihydro-1H-isoquinolin-2-yl]ethanone **4** (16.4 mg, 25% yield) as a white solid.

Basic LCMS Method 3 (ES⁺): 484/486/488 (M+H)⁺, 98% purity.

Acid LCMS Method 2 (ES⁺): 484/486/488 (M+H)⁺, 99% purity.

¹H NMR (400 MHz, DMSO-*d*₆) δ 13.46 (s, 1H), 7.93 (m, 1H), 7.68 (s, 1H), 7.54 - 7.43 (m, 2H), 7.34 - 7.04 (m, 3H), 5.35 (m, 0.3H), 5.08 (q, J = 6.3 Hz, 0.7H), 4.94 (t, J = 5.3 Hz, 0.7H), 4.70 - 4.26 (m, 3.3H), 3.90 (d, J = 5.7 Hz, 3H), 3.60 - 3.39 (m, 1H), 3.18 (dd, J = 15.8, 4.6 Hz, 1H), 3.01 (dd, J = 15.8, 5.3 Hz, 0.3H), 2.84 (q, J = 9.4, 8.9 Hz, 0.7H), 1.57 (d, J = 6.5 Hz, 0.9H), 1.29 (d, J = 6.4 Hz, 2.1H).

## Claims

1. A compound of formula (I), or a pharmaceutically acceptable salt thereof, 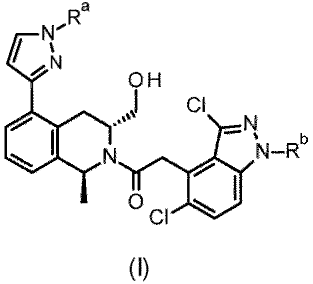 wherein R^{a} and R^{b} represent independently hydrogen or C₁₋₆ alkyl.

2. A compound of formula (I) according to Claim 1 wherein R^{a} represents hydrogen.

3. A compound of formula (I) according to Claim 1 wherein R^{a} represents C₁₋₆ alkyl.

4. A compound of formula (I) according to Claim 1 wherein R^{b} represents hydrogen.

5. A compound of formula (I) according to Claim 1 wherein R^{b} represents C₁₋₆ alkyl.

6. A compound as claimed in Claim 1 which is selected from the group consisting of:
2-(3,5-dichloro-1-methyl-indazol-4-yl)-1-[(1S,3R)-3-(hydroxymethyl)-1-methyl-5-(1-methylpyrazol-4-yl)-3,4-dihydro-1H-isoquinolin-2-yl]ethanone;
2-(3,5-dichloro-1-methyl-indazol-4-yl)-1-[(1S,3R)-3-(hydroxymethyl)-1-methyl-5-(1H-pyrazol-4-yl)-3,4-dihydro-1H-isoquinolin-2-yl]ethanone;
2-(3,5-dichloro-1H-indazol-4-yl)-1-[(1S,3R)-3-(hydroxymethyl)-1-methyl-5-(1H-pyrazol-4-yl)-3,4-dihydro-1H-isoquinolin-2-yl]ethanone; and
2-(3,5-dichloro-1H-indazol-4-yl)-1-[(1S,3R)-3-(hydroxymethyl)-1-methyl-5-(1-methylpyrazol-4-yl)-3,4-dihydro-1H-isoquinolin-2-yl]ethanone.

7. A compound of formula (I) according to any one of the preceding claims, or a pharmaceutically acceptable salt thereof, for use in therapy.

8. A compound of formula (I) according to any one of the preceding claims, or a pharmaceutically acceptable salt thereof, for use in the treatment and/or prevention of diseases and/or disorders in which D1 receptors play a role.

9. A compound of formula (I) according to any one of the preceding claims, or a pharmaceutically acceptable salt thereof, for use in the treatment and/or prevention of cognitive and negative symptoms in schizophrenia, cognitive impairment related to neuroleptic therapy, Mild Cognitive impairment (MCI), impulsivity, Attention-Deficit Hyperactivity Disorder (ADHD), Parkinson's disease and other movement disorders, dystonia, Parkinson's dementia, Huntington's disease, dementia with Lewy Body, Alzheimer's disease, drug addiction, sleep disorders, apathy, traumatic spinal cord injury or neuropathic pain.

10. A compound of formula (I) according to any one of the preceding claims, or a pharmaceutically acceptable salt thereof for use in the treatment of Parkinson's disease and other movement disorders, Alzheimer's disease, or cognitive and negative symptoms in schizophrenia.

## Patentansprüche

1. Verbindung der Formel (I) oder ein pharmazeutisch unbedenkliches Salz davon, 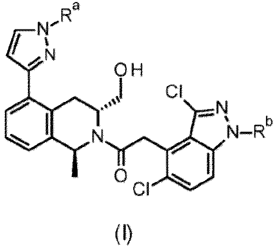 wobei R^{a} und R^{b} unabhängig für Wasserstoff oder C₁₋₆-Alkyl stehen.

2. Verbindung der Formel (I) nach Anspruch 1, wobei R^{a} für Wasserstoff steht.

3. Verbindung der Formel (I) nach Anspruch 1, wobei R^{a} für C₁₋₆-Alkyl steht.

4. Verbindung der Formel (I) nach Anspruch 1, wobei R^{b} für Wasserstoff steht.

5. Verbindung der Formel (I) nach Anspruch 1, wobei R^{b} für C₁₋₆-Alkyl steht.

6. Verbindung nach Anspruch 1, die ausgewählt ist aus der Gruppe bestehend aus:
2-(3,5-Dichlor-1-methyl-indazol-4-yl)-1-[(1S,3R)-3-(hydroxymethyl)-1-methyl-5-(1-methylpyrazol-4-yl)-3,4-dihydro-1H-isochinolin-2-yl]ethanon;
2-(3,5-Dichlor-1-methyl-indazol-4-yl)-1-[(1S,3R)-3-(hydroxymethyl)-1-methyl-5-(1H-pyrazol-4-yl)-3,4-dihydro-1H-isochinolin-2-yl]ethanon;
2-(3,5-Dichlor-1H-indazol-4-yl)-1-[(1S,3R)-3-(hydroxymethyl)-1-methyl-5-(1H-pyrazol-4-yl)-3,4-dihydro-1H-isochinolin-2-yl]ethanon; und
2-(3,5-Dichlor-1H-indazol-4-yl)-1-[(1S,3R)-3-(hydroxymethyl)-1-methyl-5-(1-methylpyrazol-4-yl)-3,4-dihydro-1H-isochinolin-2-yl]ethanon.

7. Verbindung der Formel (I) nach einem der vorhergehenden Ansprüche oder ein pharmazeutisch unbedenkliches Salz davon zur Verwendung bei der Therapie.

8. Verbindung der Formel (I) nach einem der vorhergehenden Ansprüche oder ein pharmazeutisch unbedenkliches Salz davon zur Verwendung bei der Behandlung und/oder Prävention von Erkrankungen und/oder Störungen, bei denen D1-Rezeptoren eine Rolle spielen.

9. Verbindung der Formel (I) nach einem der vorhergehenden Ansprüche oder ein pharmazeutisch unbedenkliches Salz davon zur Verwendung bei der Behandlung und/oder Prävention von kognitiven und negativen Symptomen bei Schizophrenie, mit neuroleptischer Therapie in Zusammenhang stehender kognitiver Störung, leichter kognitiver Störung (Mild Cognitive Impairment, MCI), Impulsivität, Aufmerksamkeitsdefizit-/Hyperaktivitätsstörung (Attention Disorder with Hyperactivity, ADHD), Parkinson-Krankheit und anderen Bewegungsstörungen, Dystonie, Parkinson-Demenz, Chorea Huntington, Demenz mit Lewy-Körperchen, Alzheimer-Krankheit, Drogen-/Arzneimittelsucht, Schlafstörungen, Apathie, traumatischer Rückenmarkverletzung oder neuropathischen Schmerzen.

10. Verbindung der Formel (I) nach einem der vorhergehenden Ansprüche oder ein pharmazeutisch unbedenkliches Salz davon zur Verwendung bei der Behandlung von Parkinson-Krankheit und anderen Bewegungsstörungen, Alzheimer-Krankheit oder kognitiven und negativen Symptomen bei Schizophrenie.

## Revendications

1. Composé de formule (I), ou sel pharmaceutiquement acceptable correspondant, 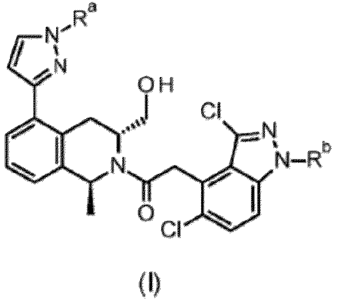 R^{a} et R^{b} représentant indépendamment hydrogène ou C₁₋₆ alkyle.

2. Composé de formule (I) selon la revendication 1, R^{a} représentant hydrogène.

3. Composé de formule (I) selon la revendication 1, R^{a} représentant C₁₋₆ alkyle.

4. Composé de formule (I) selon la revendication 1, R^{b} représentant hydrogène.

5. Composé de formule (I) selon la revendication 1, R^{b} représentant C₁₋₆ alkyle.

6. Composé selon la revendication 1 qui est choisi dans le groupe constitué par :
2-(3,5-dichloro-1-méthyl-indazol-4-yl)-1-[(1S,3R)-3-(hydroxyméthyl)-1-méthyl-5-(1-méthylpyrazol-4-yl)-3,4-dihydro-1H-isoquinoléin-2-yl]éthanone ;
2-(3,5-dichloro-1-méthyl-indazol-4-yl)-1-[(1S,3R)-3-(hydroxyméthyl)-1-méthyl-5-(1H-pyrazol-4-yl)-3,4-dihydro-1 H-isoquinoléin-2-yl]éthanone ;
2-(3,5-dichloro-1H-indazol-4-yl)-1-[(1S,3R)-3-(hydroxyméthyl)-1-méthyl-5-(1H-pyrazol-4-yl)-3,4-dihydro-1H-isoquinoléin-2-yl]éthanone ; et
2-(3,5-dichloro-1H-indazol-4-yl)-1-[(1S,3R)-3-(hydroxyméthyl)-1-méthyl-5-(1-méthylpyrazol-4-yl)-3,4-dihydro-1H-isoquinoléin-2-yl]éthanone.

7. Composé de formule (I) selon l'une quelconque des revendications précédentes, ou sel pharmaceutiquement acceptable correspondant, pour une utilisation en thérapie.

8. Composé de formule (I) selon l'une quelconque des revendications précédentes, ou sel pharmaceutiquement acceptable correspondant, pour une utilisation dans le traitement et/ou la prévention de maladies et/ou de troubles dans lesquels les récepteurs de D1 jouent un rôle.

9. Composé de formule (I) selon l'une quelconque des revendications précédentes, ou sel pharmaceutiquement acceptable correspondant, pour une utilisation dans le traitement et/ou la prévention de symptômes cognitifs et négatifs dans la schizophrénie, une déficience cognitive liée à une thérapie neuroleptique, un déficit cognitif léger (MCI), l'impulsivité, un trouble de l'hyperactivité avec déficit de l'attention (TDAH), la maladie de Parkinson et d'autres troubles du mouvement, la dystonie, la démence de Parkinson, la maladie de Huntington, la démence avec corps de Lewy, la maladie d'Alzheimer, la toxicomanie, les troubles du sommeil, l'apathie, une lésion de la moelle épinière traumatique ou une douleur neuropathique.

10. Composé de formule (I) selon l'une quelconque des revendications précédentes, ou sel pharmaceutiquement acceptable correspondant, pour une utilisation dans le traitement de la maladie de Parkinson et d'autres troubles du mouvement, la maladie d'Alzheimer ou des symptômes cognitifs et négatifs dans la schizophrénie.
